(19) **Europäisches Patentamt / European Patent Office / Office européen des brevets**

(11) **EP 3 860 555 B1**

(12) **EUROPEAN PATENT SPECIFICATION**

(45) Date of publication and mention
of the grant of the patent:
**15.01.2025  Bulletin 2025/03**

(21) Application number: **19783002.9**

(22) Date of filing: **02.10.2019**

(51) International Patent Classification (IPC):
*A61K 8/35* (2006.01)        *A61K 8/368* (2006.01)
*A61K 8/37* (2006.01)        *A61K 8/39* (2006.01)
*A61K 8/41* (2006.01)        *A61K 8/49* (2006.01)
*A61K 8/58* (2006.01)        *A61Q 17/04* (2006.01)

(52) Cooperative Patent Classification (CPC):
**A61K 8/585; A61K 8/35; A61K 8/368; A61K 8/37;
A61K 8/39; A61K 8/415; A61K 8/496;
A61K 8/4966; A61Q 17/04**

(86) International application number:
**PCT/EP2019/076712**

(87) International publication number:
**WO 2020/070193 (09.04.2020 Gazette 2020/15)**

(54) **SUNSCREEN COMPOSITIONS COMPRISING DROMETRIZOLE TRISILOXANE TO REDUCE FABRIC STAINING**

SONNENSCHUTZZUSAMMENSETZUNGEN MIT DROMETRIZOL-TRISILOXAN ZUR REDUZIERUNG DER STOFFEINFÄRBUNG

COMPOSITIONS D'ÉCRAN SOLAIRE COMPRENANT DU DROMÉTRIZOLE TRISILOXANE AFIN DE RÉDUIRE LA COLORATION DE TISSU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.10.2018  EP 18198923**

(43) Date of publication of application:
**11.08.2021  Bulletin 2021/32**

(73) Proprietor: **BASF SE
67056 Ludwigshafen am Rhein (DE)**

(72) Inventors:
• **SOHN, Myriam
79639 Grenzach-Wyhlen (DE)**
• **KRUS, Stanislaw
79639 Grenzach-Wyhlen (DE)**
• **LINDEMANN, Brigitte
79639 Grenzach-Wyhlen (DE)**
• **GRUMELARD, Julie
79639 Grenzach-Wyhlen (DE)**

(74) Representative: **BASF IP Association
BASF SE
GBI - Z078
67056 Ludwigshafen (DE)**

(56) References cited:
**AU-A1- 2018 200 455     US-A1- 2009 099 075**

• **DATABASE GNPD [online] MINTEL; 19 June 2017 (2017-06-19), ANONYMOUS: "Waterlover Sun Milk SPF 50", XP055545366, retrieved from https://www.gnpd.com/sinatra/recordpage/4773799/ Database accession no. 4773799**
• **DATABASE GNPD [online] MINTEL; 14 August 2018 (2018-08-14), ANONYMOUS: "Sunscreen Broad Spectrum SPF 50+ PA++++", XP055545361, retrieved from https://www.gnpd.com/sinatra/recordpage/5866465/ Database accession no. 5866465**

**(Cont. next page)**

- DATABASE GNPD [online] MINTEL; 12 July 2017 (2017-07-12), ANONYMOUS: "Waterlover Sun Milk SPF 15", XP055545364, retrieved from https://www.gnpd. com/sinatra/recordpage/4927473/ Database accession no. 4927473
- DATABASE GNPD [online] MINTEL; 14 August 2018 (2018-08-14), ANONYMOUS: "Sunscreen Broad Spectrum SPF 50+ PA++++", XP055545361, retrieved from www.gnpd.com Database accession no. 5866465
- DATABASE GNPD [online] MINTEL; 12 July 2017 (2017-07-12), ANONYMOUS: "Waterlover Sun Milk SPF 15", XP055545364, retrieved from www.gnpd.com Database accession no. 4927473
- DATABASE GNPD [online] MINTEL; 19 June 2017 (2017-06-19), ANONYMOUS: "Waterlover Sun Milk SPF 50", XP055545366, retrieved from www.gnpd.com Database accession no. 4773799

**Description**

The present invention relates to the use of drometrizole trisiloxane to reduce fabric staining of sunscreen compositions. Furthermore, the present invention relates to the use of drometrizole trisiloxane to facilitate the washability of sunscreen compositions from textiles. Furthermore, the present invention relates to a sunscreen composition comprising inter alia drometrizole trisiloxane.

UV radiation causes harmful effects on the human skin. Beside the acute effect of sunburn of the skin, UV radiation is also known to increase the risk of skin cancer. Furthermore, long time exposure to UV-A and UV-B light can cause phototoxic and photoallergenic reactions on the skin and can accelerate skin aging.

To protect the human skin from UV radiation, various sun protecting UV filters (also referred to as UV absorbers) exist including UV-A filter, UV-B filter and broadband filters. These filters are added to sunscreen or cosmetic compositions. The UV filters are either organic or inorganic compounds, which have a high absorption efficacy in the UV-light range. In general, UV light can be divided into UV-A radiation and UV-B radiation. Depending on the position of the absorption maxima, UV-filters are divided into UV-A and UV-B filters. In case an UV-filter absorbs both, UV-A and UV-B light, it is referred to as a broadband absorber.

Since 2006, the EU commission has recommended that all sunscreen or cosmetic compositions should have an UV-A protection factor, which is at least one third of the labelled sun protection factor (SPF), wherein the sun protection factor refers mainly to the UV-B protection.

However, the use of UV-A filters and broadband filters in sunscreen or cosmetic compositions has certain disadvantages. In particular, sunscreen or cosmetic compositions can adhere on fabrics or textiles and cause fabric staining. In view of the low solubility of the filters in water, the resulting stains are hard to remove during washing of the fabrics.

Accordingly, it is desired to reduce fabric staining and/or facilitate the washability of sunscreen compositions from textiles without reducing the UV-A protection.

DE 10 2014 207 919 A1 discloses sunscreen compositions with reduced fabric staining comprising bis-ethylhexyloxyphenol methoxyphenyl triazine in combination with certain chelating agents.

DE 10 2014 207 916 A1 discloses sunscreen compositions with reduced fabric staining comprising butyl methoxydibenzoylmethane in combination with certain chelating agents.

In contrast, DE 10 2014 207 935 A1 is directed to sunscreen compositions in general, which show in combination with chelating agents less fabric staining.

DE 10 2014 207 924 A1 the UV-filter diethylamino hydroxybenzoyl hexyl benzoate in combination with certain chelating agents in sunscreen composition for reduced fabric staining.

EP 2 939 710 A1 discloses a method to reduce substantivity of cloth staining caused by sunscreens, whereby the focus is on cosmetic and/or pharmaceutical preparations comprising disodium phenyl benzimidazole tetrasulfonic acid and/or its salts.

EP 3 150 190 A1 discloses sunscreen compositions for reduced fabric staining comprising pirocton and or salts thereof.

EP 3 153 151 A1 is directed to sunscreen compositions which show reduced fabric staining, whereby the sunscreen composition comprises tert-butyl-methoxydibenzoylmethane and at least two additives.

EP 3 150 258 A1 discloses sunscreen compositions comprising bis-ethylhexyloxyphenol methoxyphenyl triazine and pirocton for reduced fabric staining.

EP 3 150 189 is directed to sunscreen compositions comprising diethylamino hydroxybenzoyl hexyl benzoate in combination with pirocton to reduce fabric staining.

EP 3 354 253 A2 discloses the use of diethylamino hydroxybenzoyl hexyl benzoate for the reduction of fabric staining of a cosmetic composition.

However, there remains a need for sunscreen compositions, which are further improved regarding the problem of fabric staining and the washability from textiles without reducing the UV-A protection.

Therefore, it has been an object of the present invention to provide sunscreen compositions with improved fabric staining and washability properties. In this connection, it has been another object to provide sunscreen compositions, which nevertheless provide satisfying UV-A protection in addition to UV-B protection. It has been another object of the present invention to provide an UV filter providing UV-A protection, which is suitable for use in sunscreen compositions in order to reduce fabric staining, and/or to facilitate the washability of sunscreen compositions from textiles.

It has surprisingly been found that at least one of these objects can be achieved by the use of 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) in sunscreen compositions.

In particular, the inventors of the present application found out that by using drometrizole trisiloxane fabric staining of sunscreen compositions can be reduced and the washability of the stains from textiles can be facilitated.

Thus, according to one embodiment, the present invention relates to the use of 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) to reduce fabric staining of sunscreen compositions.

In another embodiment, the present invention relates to the use of 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) to facilitate the washability of sunscreen compositions.

In a preferred embodiment of said uses, the sunscreen composition has an UV-A protection, which is at least one third of the sun protection factor of the sunscreen composition, and/or shows a critical wavelength of at least 370 nm.

In another preferred embodiment of said uses, the sunscreen composition comprises at least one further UV-A filter.

In a more preferred embodiment of said uses, the at least one further UV-A filter is selected from the group consisting of triazine derivatives, benzotriazole derivatives, benzophenone derivatives, dibenzoylmethane derivatives, camphor derivatives and combinations thereof.

In an even more preferred embodiment of said uses, the at least one UV-A filter is selected from the group consisting of 4-(tert.-butyl)-4'-methoxydibenzoylmethane (INCI butyl methoxydibenzoylmethane), hexyl 2-[4-(diethylamino)-2-hydroxybenzoyl]benzoate (INCI diethylamino hydroxybenzoyl hexyl benzoate), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), and combinations thereof.

In another preferred embodiment of said uses, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 0.2 to 15 % by weight, preferably from 0.5 to 5 % by weight, more preferably from 1 to 3 % by weight based on the total weight of the sunscreen composition.

In another preferred embodiment of said uses, the sunscreen composition comprises at least one further UV-B filter.

In another preferred embodiment of said uses, the at least one further UV-B filter is selected from the group consisting of triazine derivatives, cinnamate derivatives, salicylate derivatives, malonate derivatives, benzimidazole derivatives and combinations thereof.

In a more preferred embodiment of said uses, the at least one UV-B filter is selected from the group consisting of 4,4',4"-(1,3,5-triazin-2,4,6-triyltriimino)tris-benzoic acid-tris(2-ethylhexyl)ester (INCI ethylhexyl triazone), (RS)-2-ethylhexyl-2-hydroxybenzoate (INCI ethylhexyl salicylate), 3,3,5-trimethyl-cyclohexyl salicylate (homosalate), 2-ethylhexyl 2-cyano-3,3-diphenyl-2-propenoate (INCI octocrylene), (RS)-2-ethylhexyl-(2E)-3-(4-methoxyphenyl)prop-2-enoate (INCI ethylhexyl methoxycinnamate), malonate derivatives such as dimethicone diethyl benzalmalonate, diethylhexyl butamido triazone, and combinations thereof.

In another preferred embodiment of said uses, the sunscreen composition further comprises at least one water soluble UV filter.

In a more preferred embodiment, the water soluble UV filter is selected from the group consisting of [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1-bicyclo[2.2.1]heptanyl]methanesulfonic acid (INCI terephthalylidene dicamphor sulfonic acid), 2-phenyl-1H-benzimidazole-5-sulfonic acid (INCI phenylbenzimidazole sulfonic acid), disodium phenyl dibenzimidazole tetrasulfonate, and combinations thereof.

In another preferred embodiment of said uses, the sunscreen composition further comprises at least one particulate filter. Preferably, the at least one particulate UV filter is an organic particulate UV filter, an inorganic UV filter, or a combination thereof.

In a more preferred embodiment, the at least one organic particulate UV filter is selected from the group consisting of 2,2'-methylene bis[6-(2H-benzotriazol-2-yl)-4-(1,1,3,3-tetramethylbutyl)phenol] (INCI methylene bis-benzotriazolyl tetramethylbutylphenol), 2,4,6-tris(biphenyl-4-yl)-1,3,5-triazine (INCI trisbiphenyl triazine), 1,1'-(1,4-piperazinediyl)bis[1-[2-[4-(diethylamino)-2-hydroxybenzoyl]phenyl]-methanone (INCI bis-(diethylaminohydroxybenzoyl benzoyl) piperazine), 5,6,5',6'-tetraphenyl-3-3'-(1,4-phenylene)bis(1,2,4-triazine) (INCI phenylene bis-diphenyltriazine), and combinations thereof.

In another more preferred embodiment, the at least one inorganic particulate UV filter is selected from the group consisting of titanium dioxide, zinc oxide, and combinations thereof.

In another embodiment, the invention relates to a sunscreen composition comprising

(i) 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane),

(ii) hexyl 2-[4-(diethylamino)-2-hydroxybenzoyl]benzoate (INCI diethylamino hydroxybenzoyl hexyl benzoate),

(iii) 4,4',4"-(1,3,5-triazin-2,4,6-triyltriimino)tris-benzoic acid-tris(2-ethylhexyl)ester (INCI ethylhexyl triazone), and

(iv) (RS)-2-ethylhexyl-2-hydroxybenzoate (INCI ethylhexyl salicylate);

wherein the sunscreen composition comprises at least one further UV filter, wherein the at least one further UV filter is 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine); and wherein the at least one further UV filter 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine) is present in the sunscreen composition in an amount of from 0.1 to 2.8 % by weight, based on the total weight of the sunscreen composition.

In a preferred embodiment of said sunscreen composition, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 0.1 to 15 % by weight, preferably from 0.5 to 5 % by weight, more preferably from 1 to 3 % by weight based on the total weight of the sunscreen composition.

In another preferred embodiment of said sunscreen composition, the hexyl 2-[4-(diethylamino)-2-hydroxybenzoyl]benzoate (INCI diethylamino hydroxybenzoyl hexyl benzoate) is present in an amount of from 0.1 to 10 % by weight, preferably from 1 to 7 % by weight based on the total weight of the sunscreen composition.

In another preferred embodiment of said sunscreen composition, the 4,4',4"-(1,3,5-triazin-2,4,6-triyltriimino)tris-benzoic acid-tris(2-ethylhexyl)ester (INCI ethylhexyl triazone) is present in an amount of from 0.1 to 5 % by weight, preferably from 0.5 to 3 % by weight based on the total weight of the sunscreen composition.

In another preferred embodiment of said sunscreen composition, the (RS)-2-ethylhexyl-2-hydroxybenzoate (INCI ethylhexyl salicylate) is present in an amount of from 0.1 to 5 % by weight based on the total weight of the sunscreen composition.

In another aspect the present invention relates to a method to reduce fabric staining of sunscreen compositions, wherein the method comprises adding 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) to a sunscreen composition.

In another aspect the present invention refers to a method to facilitate the washability of sunscreen compositions, wherein the method comprises adding 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) to a sunscreen composition.

In a preferred embodiment of said method, the sunscreen composition has an UV-A protection, which is at least one third of the sun protection factor of the sunscreen composition, and/or shows a critical wavelength of at least 370 nm.

In another preferred embodiment of said method, the sunscreen composition comprises at least one further UV-A filter.

In another preferred embodiment of said method, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 0.2 to 15 % by weight, preferably from 0.5 to 5 % by weight, more preferably from 1 to 3 % by weight based on the total weight of the sunscreen composition.

In another preferred embodiment of said method, the sunscreen composition comprises at least one further UV-B filter.

In a more preferred embodiment of said method, the at least one further UV-B filter is selected from the group consisting of triazine derivatives, cinnamate derivatives, salicylate derivatives, malonate derivatives, benzimidazole derivatives and combinations thereof.

In an even more preferred embodiment of said method, the at least one UV-B filter is selected from the group consisting of 4,4',4"-(1,3,5-triazin-2,4,6-triyltriimino)tris-benzoic acid-tris(2-ethylhexyl)ester (INCI ethylhexyl triazone), (RS)-2-ethylhexyl-2-hydroxybenzoate (INCI ethylhexyl salicylate), 3,3,5-trimethyl-cyclohexyl salicylate (homosalate), 2-ethylhexyl 2-cyano-3,3-diphenyl-2-propenoate (INCI octocrylene), (RS)-2-ethylhexyl-(2E)-3-(4-methoxyphenyl)prop-2-enoate (INCI ethylhexyl methoxycinnamate), malonate derivatives such as dimethicone diethyl benzalmalonate, diethylhexyl butamido triazone, and combinations thereof.

In another preferred embodiment of said method, the sunscreen composition further comprises at least one water soluble UV filter.

In a more preferred embodiment, the water soluble UV filter is selected from the group consisting of [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1-bicyclo[2.2.1]heptanyl]methanesulfonic acid (INCI terephthalylidene dicamphor sulfonic acid), 2-phenyl-1H-benzimidazole-5-sulfonic acid (INCI phenylbenzimidazole sulfonic acid), disodium phenyl dibenzimidazole tetrasulfonate, and combinations thereof.

In another preferred embodiment of said method, the sunscreen composition further comprises at least one particulate filter. Preferably, the at least one particulate UV filter is an organic particulate UV filter, an inorganic UV filter, or a combination thereof.

In a more preferred embodiment, the at least one organic particulate UV filter is selected from the group consisting of 2,2'-methylene bis[6-(2H-benzotriazol-2-yl)-4-(1,1,3,3-tetramethylbutyl)phenol] (INCI methylene bis-benzotriazolyl tetramethylbutylphenol), 2,4,6-tris(biphenyl-4-yl)-1,3,5-triazine (INCI trisbiphenyl triazine), 1,1'-(1,4-piperazinediyl)bis[1-[2-4-(diethylamino)-2-hydroxybenzoyl]phenyl]-methanone (INCI bis-(diethylaminohydroxybenzoyl benzoyl) piperazine), 5,6,5',6'-tetraphenyl-3-3'-(1,4-phenylene)bis(1,2,4-triazine) (INCI phenylene bis-diphenyltriazine), and combinations thereof.

In another more preferred embodiment, the at least one inorganic particulate UV filter is selected from the group consisting of titanium dioxide, zinc oxide, and combinations thereof.

Before describing preferred embodiments of the present invention in detail, definitions important for understanding the present invention are given.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of $\pm 20$ %, preferably $\pm 15$ %, more preferably $\pm 10$ %, and even more preferably $\pm 5$ %. It is to be understood that the term "comprising" is not limiting. For the purposes of the present invention, the term "consisting of" is considered to be a preferred embodiment of the term "comprising of". If hereinafter a group is defined to comprise at least a certain number of embodiments, this is meant to also encompass a group, which preferably consists of these embodiments only. Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The term "sunscreen composition" refers to any topical product, which reflects and/or absorbs certain parts of UV radiation. Thus, the term "sunscreen composition" is to be understood as not only including sunscreen compositions, but also any cosmetic compositions that provide UV protection. The term "topical product" refers to a product that is applied to the skin and can refer, e.g., to sprays, lotions, creams, oils, or gels. The sunscreen composition may comprise one or more active agents, e.g., organic or inorganic UV filters, as well as other ingredients or additives, e.g., emulsifiers, emollients, viscosity regulators, stabilizers, preservatives, or fragrances.

The term "fabric staining" as used herein refers to the observation that sunscreen compositions, in particular those providing good UV-A protection, leave yellow on textiles. This is undesired because the sunscreen composition is often in contact with textiles during or after the application to the human skin, so that stains will be observed on the textiles. Staining of fabrics can be measured by the L*a*b as outlined in detail below.

The term "washability" as used herein describes to which extent stains on textiles caused by sunscreen compositions can be removed from the textile after a certain number of washing cycles. In order to assess the washability, fabric staining is measured before and after one or more washing cycles. A washing cycle is carried out at a temperature of from 30°C to 50 °C with a commercially available liquid washing gel. Typically, the difference regarding fabric staining after 1 to 3 washing cycles is determined.

The term "UV-filter" as used herein refers to organic or inorganic compounds, which can absorb or reflect UV radiation caused by sunlight. UV-filter can be classified based on their UV protection curve as UV-A, UV-B or broadband filters. In the context of the present application, broadband filters may be listed as UV-A filters, as they also provide UV-A protection. In other words, preferred UV-A filters also include broadband filters.

As used herein, 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane), also referred to as DTS, is an UV filter belonging to the group of benzotriazole derivatives. It is a broadband UV filter with two absorption peaks, one at 303 nm (UVB) and one at 344 nm (UVA). Further UV filters are listed below.

The term "sun protection factor (SPF)" as used herein indicates how well the skin is protected by a sunscreen composition mainly from UV-B radiation. In particular, the factor indicates how much longer the protected skin may be exposed to the sun without getting a sunburn in comparison to untreated skin. For example, if a sunscreen composition with an SPF of 15 is evenly applied to the skin of a person usually getting a sunburn after 10 minutes in the sun, the sunscreen allows the skilled person to stay in the sun 15 times longer. In other words, SPF 15 means that 1/15 of the burning UV radiation will reach the skin, assuming sunscreen is applied evenly at a thick dosage of 2 milligrams per square centimeter ($mg/cm^2$).

The term "critical wavelength" is defined as the wavelength at which the area under the UV protection curve (% protection versus wavelength) represents 90 % of the total area under the curve in the UV region (290-400 nm). For example, a critical wavelength of 370 nm indicates that the protection of the sunscreen composition is not limited to the wavelengths of UV-B, i.e. wavelengths from 290-320 nm, but extends to 370 nm in such a way that 90 % of the total area under the protective curve in the UV region are reached at 370 nm.

The definition of "broadband" protection (also referred to as broad-spectrum or broad protection) is based on the "critical wavelength". For broadband coverage, UV-B and UV-A protection must be provided. According to the US requirements, a critical wavelength of at least 370 nm is required for achieving broad spectrum protection.

Preferred embodiments regarding the uses of 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) as well as methods comprising its application according to the present invention are described hereinafter. It is to be understood that the preferred embodiments of the invention are preferred alone or in combination with each other.

As indicated above, the present invention relates in one embodiment to the use of 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) to reduce fabric staining of sunscreen compositions.

Fabric staining of sunscreen compositions is preferably measured by the so-called L*a*b method mentioned above. With this method, the color difference between two samples can be identified based on numerical values. The advantage of this method is that it is not dependent on the device used for measuring the colors. Instead, the color difference can be determined based on numerical values for different colors. As used herein, the color difference between a sunscreen composition stain on a textile and the textile alone is determined in order to determine fabric staining of a sunscreen composition. Different sunscreen compositions and their fabric staining properties can thus be measured by comparing the color of their stains on a textile with the color of the textile alone. The lower the color difference is, the less fabric staining is observed. Similarly, the color difference between a sunscreen composition stain on a textile after one or more washing cycles and the textile alone can be determined in order to determine a potential reduction of fabric staining after washing, i.e. the washability. The color difference is indicated in absolute color coordinates and can be referred to as Delta $\Delta$. First, the colors of the two samples are measured. Then, the difference can be calculated using the resulting colorimetric values, the CIE L*a*b coordinates. The L*a*b color space, defined by the Commission Internationale de l'Eclairage (CIE), was modeled after a color-opponent theory. This theory states that two colors cannot be red and green at the same time or yellow and blue at the same time. The following formula is used to determine the total color difference between two samples:

$$\Delta E^* = [\Delta L^{*2} + \Delta a^{*2} + \Delta b^{*2}]^{1/2}$$

In this regard, L* indicates lightness, a* is the red/green coordinate, and b* is the yellow/blue coordinate. Delta values for L* ($\Delta$L*), a* ($\Delta$a*), and b* ($\Delta$b*) can be positive or negative. The total difference, given as $\Delta$E* is always positive.

$\Delta$L* (L* sample1 - L* sample 2) = difference in lightness and darkness (+ is lighter, - is darker).
$\Delta$a* (a* sample 1 - a* sample 2) = difference in red and green (+ is redder, - is greener)
$\Delta$b* (b* sample 1 - b* sample 2) = difference in yellow and green (+ is yellower, - is greener)
$\Delta$E* = total color difference.

In order to determine fabric staining according to the present invention, the color of the stain on a textile is compared to the color of the textile alone. Preferably, a white textile is used. In case the stain is only yellow, $\Delta E^* \cong \Delta b^*$, wherein the visibility of the sunscreen composition stain is expressed by $\Delta$b*. As the color of a sunscreen composition stain is typically yellow, $\Delta$E* is considered to be identical to $\Delta$b* for the purpose of the present invention.

In another embodiment, the present invention relates to the use of 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) to facilitate the washability of sunscreen compositions from textiles. As used herein, the washability refers to the extent to which sunscreen composition stains on textiles can be removed by washing. The washability, i.e. the removability, of a sunscreen composition stain can be determined by determining the color difference of the stain according to the L*a*b* method described above before and after one or more washing cycles.

In connection with the present invention, the following preferred embodiments regarding the sunscreen composition are relevant.

In a preferred embodiment of the present invention, the sunscreen composition has an UV-A protection, which is at least one third of the sun protection factor as defined above. For example, if the sun protection factor of a sunscreen composition is 30, the UV-A protection of the given sunscreen composition has to be at least 10. In another preferred embodiment of the invention, the UV-A protection of the sunscreen composition has to show a critical wavelength of at least 370 nm. In another preferred embodiment of the invention, the sunscreen composition has an UV-A protection, which is at least one third of the sun protection factor as defined above, and also shows a critical wavelength of at least 370 nm.

A skilled person is aware that a sunscreen composition providing good UV-A protection according to the above criteria requires the presence of at least one UV-A filter. As used herein, 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane), also referred to as DTS, serves as UV-A filter. In addition, at least one further UV-A filter may be present. In this context, it has surprisingly been found by the inventors of the present invention that although higher amounts of UV-A filters are required to reach a sufficient UV-A protection of the sunscreen composition, the use of DTS reduces fabric staining of the sunscreen composition according to the present invention.

In a preferred embodiment of the present invention, the sunscreen composition comprises at least one further UV-A filter. In this context, at least one further UV-A filter means from 1 to 3 further UV-A filters, preferably 1 or 2 further UV-A filters. Furthermore, it is to be understood that the term UV-A filter means any UV filter absorbing in the UV-A range. This means that also broadband filters are encompassed by the expression UV-A filter, since they absorb and/or reflect UV-A and UV-B radiation.

In a more preferred embodiment, the at least one further UV-A filter is selected from the group consisting of triazine derivatives, benzotriazole derivatives, benzophenone derivatives, dibenzoylmethane derivatives, camphor derivatives, and combinations thereof.

In an even more preferred embodiment, the at least one further UV-A filter is selected from the group consisting of 4-(tert.-butyl)-4'-methoxydibenzoylmethane (INCI butyl methoxydibenzoylmethane), hexyl 2-[4-(diethylamino)-2-hydroxybenzoyl]benzoate (INCI diethylamino hydroxybenzoyl hexyl benzoate), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), and combinations thereof.

In another preferred embodiment, the sunscreen composition further comprises at least one water soluble UV filter.

In a more preferred embodiment, the water soluble UV filter is selected from the group consisting of [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1-bicyclo[2.2.1]heptanyl]methanesulfonic acid (INCI terephthalylidene dicamphor sulfonic acid), 2-phenyl-1H-benzimidazole-5-sulfonic acid (INCI phenylbenzimidazole sulfonic acid), disodium phenyl dibenzimidazole tetrasulfonate, and combinations thereof.

In another preferred embodiment, the sunscreen composition further comprises at least one particulate filter. Preferably, the at least one particulate UV filter is an organic particulate UV filter, an inorganic UV filter, or a combination thereof.

In a more preferred embodiment, the at least one organic particulate UV filter is selected from the group wherein the at least one organic particulate UV-filter is selected from the group consisting of 2,2'-methylene bis[6-(2H-benzotriazol-2-yl)-4-(1,1,3,3-tetramethylbutyl)phenol] (INCI methylene bis-benzotriazolyl tetramethylbutylphenol), 2,4,6-tris(biphenyl-4-yl)-1,3,5-triazine (INCI trisbiphenyl triazine), 1,1'-(1,4-piperazinediyl)bis[1-[2-[4-(diethylamino)-2-hydroxybenzoyl]phenyl]-methanone (INCI bis-(diethylaminohydroxybenzoyl benzoyl) piperazine), 5,6,5',6'-tetraphenyl-3-3'-(1,4-phenylene)bis(1,2,4-triazine) (INCI phenylene bis-diphenyltriazine), and combinations thereof.

In another more preferred embodiment, the at least one inorganic particulate UV filter is selected from the group consisting of titanium dioxide, zinc oxide, and combinations thereof.

Suitable amounts of a further UV-A filter are in the range of from 0.1 to 10 % by weight, preferably from 1 to 7 % by weight based on the total weight of the sunscreen composition. However, it is preferred that the amount of a further UV-A-filter is as low as possible in order to avoid fabric staining. It can therefore be preferred that the amount of a further UV-A filter is in the range of from 0.1 to 5 % by weight based on the total weight of the sunscreen composition.

Suitable amounts of particulate UV filters are in the range of from 0.1 to 10 % by weight, preferably from 0.5 to 7 % by weight, based on the total weight of the sunscreen composition.

Suitable amounts of water soluble UV filters are in the range of from 0.1 to 6 % by weight, preferably from 0.5 to 3 % by weight, based on the total weight of the sunscreen composition.

In a preferred embodiment of the present invention, the sunscreen composition additionally comprises at least one further UV-B filter. In this context, at least one further UV-A filter means from 1 to 3 further UV-B filters, preferably 1 or 2 further UV-B filters. Furthermore, it is to be understood that the term UV-B filter means any UV filter absorbing in the UV-B range. This means that also broadband filters are encompassed by the expression UV-B filter, since they absorb and/or reflect UV-A and UV-B radiation.

In a more preferred embodiment, the at least one further UV-B filter is selected from the group consisting of triazine derivatives, cinnamate derivatives, salicylate derivatives, malonate derivatives, benzimidazole derivatives, and combinations thereof.

In a particularly preferred embodiment, the at least one further UV-B filter is selected from the group consisting of 4,4',4"-(1,3,5-triazin-2,4,6-triyltriimino)tris-benzoic acid-tris(2-ethylhexyl)ester (INCI ethylhexyl triazone), (RS)-2-ethylhexyl-2-hydroxybenzoate (INCI ethylhexyl salicylate), 3,3,5-trimethyl-cyclohexyl salicylate (homosalate), 2-ethylhexyl 2-cyano-3,3-diphenyl-2-propenoate (INCI octocrylene), (RS)-2-ethylhexyl-(2E)-3-(4-methoxyphenyl)prop-2-enoate (INCI ethylhexyl methoxycinnamate), malonate derivatives such as dimethicone diethyl benzalmalonate, diethylhexyl butamido triazone, and combinations thereof.

Suitable amounts of a further UV-B filter are in the range of from 0.1 to 10 % by weight based on the total weight of the sunscreen composition.

In a preferred embodiment of the uses of the present invention, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 0.2 to 15 % by weight based on the total weight of the sunscreen composition. In a more preferred embodiment, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in an amount of from 0.3 to 10 % by weight based on the total weight of the sunscreen composition. In an even more preferred embodiment, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in an amount of from 0.5 to 5 % by weight based on the total weight of the sunscreen composition. In a particularly preferred embodiment, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in an amount of from 1 to 3 % by weight based on the total weight of the sunscreen composition.

In connection with the above preferred embodiments, it is to be understood that the total weight of the sunscreen composition refers to the sunscreen composition as defined above, wherein the sunscreen composition may comprise at least one additive.

In one embodiment, the at least one additive is selected from the group consisting of emulsifier, emollients, viscosity regulators (thickeners), sensory enhancers, adjuvants, preservatives, and combinations thereof.

Preferred emulsifiers include

- glucose derivatives such as cetearyl glucoside, arachidyl glucoside, lauryl glucoside, polyglyceryl-3 methylglucose distearate, methyl glucose sesquistearate;
- sucrose derivative such as sucrose polystearate, sucrose palmitate;
- sorbitol derivatives such as polysorbate-n, PEG-10 sorbitan laurate;
- fatty alcohol polyglycolethers and fatty acid polyglycolethers such as ceteareth-20, beheneth-25, steareth-2, PEG-100 stearate;
- glycerides of fatty acids such as glyceryl stearate, glyceryl oleate;
- glumatic acid derivatives such as sodium stearoyl glutamate;
- sulfosuccinic acid derivatives such as disodium Cetearyl sulfosuccinate;
- phosphoric acid derivatives such as potassium cetyl phosphate;
- fatty acid esters of polyglyceryl such as polyglyceryl-3-diisostearate, polyglyceryl-2-dipolyhydroxystearate;
- oxyalkenylated organomodified silicone / polysiloxane / polyalkyl / polyether copolymers and derivatives.

Preferred emollients include

- esters of linear or branched fatty acids with linear or branched fatty alcohols such as propylheptyl caprylate, coco caprylate, isopropyl myristate, ethylhexyl palmitate;
- esters of aromatic carboxylic acids with linear or branched fatty alcohols such as $C_{12}$-$C_{15}$-alkyl benzoate, ethylhexyl benzoate, phenethyl benzoate;
- dicarboxylic acid esters with linear or branched alcohols such as dibutyl adipate, dicaprylyl carbonate, diisopropyl sebacate;
- esters of hydroxycarboxylic acids with linear or branched fatty alcohols;
- esters of linear or branched fatty acids with polyhydric alcohol such as butylene glycol dicaprylate/dicaprate;
- mono-, di-, tri-glycerides based on $C_6$-$C_{18}$ fatty acids such as caprylic / capric triglycerides, coco glycerides;
- guerbet alcohols such as octyldodecynol;
- hydrocarbons such as hydrogenated polyisobutene, mineral oil, squalene, isohexadecane;
- ethers such as dicaprylyl ether;
- silicone derivatives (organomodified polysiloxanes) such as dimethylpolysiloxane, cyclic silicones.

Preferred thickeners include

- fatty alcohols such as cetyl alcohol, cetearyl alcohol, stearyl alcohol;
- fatty acids such as stearic acid;
- fatty acid esters such as myristyl stearate;
- waxes such as beeswax, carnauba wax, microcrystalline wax, ceresin, ozocerite;
- polysaccharides or derivatives such as xanthan gum, guar gum, agar gum, alginates, gellan gum, carraghenan;
- polyacrylates or homopolymers of reticulated acrylic acids or polyacrylamides such as carbomers, acrylate copolymers, acrylate / $C_{10}$-$C_{30}$-alkyl acrylate crosspolymer, acrylate / beheneth-25 methacrylate copolymer;
- silicate derivatives such as magnesium silicates;
- cellulose derivatives such as hydroxypropyl cellulose.

Preferred sensory enhancers include

- polyamide derivatives such as nylon-12;
- polymethyl methacrylates;

- silica;
- mica;
- polymethylsilsesquioxane;
- polyethylene;
- starch derivatives such as aluminum starch octenylsuccinate;
- dimethicone derivatives;
- boron nitride;
- HDI / trimethylol hexyllactone crosspolymer.

Preferred adjuvants include

- tocopherol derivatives;
- retinol derivatives;
- ascorbic acid derivatives;
- bisabolol;
- allantoin;
- panthenol;
- chelating agents (EDTA, EDDS, EGTA, phytic acid, piroctone olamine);
- ethylhexyl glycerin;
- caprylyl glycol;
- hydroxyacetophenone;
- caprylhydroxymic acid;
- propellants such as propane, butane, isobutene, dimethyl ether;
- styrene / PVP or styrene acrylamide copolymers;
- insect repellants such as butylacetylaminopropionate.

Preferred preservatives include

- paraben derivatives;
- benzyl alcohol;
- phenoxyethanol;
- zingerone.

In connection with the above preferred embodiments, it is to be understood that if the sunscreen composition comprises two or more additives, combinations of the additives as defined above are also part of the invention.

In connection with the above preferred and particularly preferred embodiments, it is to be understood that the sunscreen composition may further comprise water. In case water is present in the sunscreen composition, the sunscreen composition can be an oil in water emulsion (O/W emulsion) or a water in oil emulsion (W/O emulsion).

In another aspect, the present invention refers to a sunscreen composition comprising

(i) 2-(2H-benzotriazol-2-yl)-4-methyl-6-[2-methyl-3-[1,3,3,3-tetramethyl-1-[(trimethylsilyl)oxy]-1-disiloxanyl]propyl] phenol (INCI drometrizole trisiloxane),
(ii) hexyl 2-[4-(diethylamino)-2-hydroxybenzoyl]benzoate (INCI diethylamino hydroxybenzoyl hexyl benzoate),
(iii) 4,4',4"-(1,3,5-triazin-2,4,6-triyltriimino)tris-benzoic acid-tris(2-ethylhexyl)ester (INCI ethylhexyl triazone), and
(iv) (RS)-2-ethylhexyl-2-hydroxybenzoate (INCI ethylhexyl salicylate);

wherein the sunscreen composition comprises at least one further UV filter, wherein the at least one further UV filter is 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine); and wherein the at least one further UV filter 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine) is present in the sunscreen composition in an amount of from 0.1 to 2.8 % by weight, based on the total weight of the sunscreen composition.

Hexyl 2-[4-(diethylamino)-2-hydroxybenzoyl]benzoate (INCI diethylamino hydroxybenzoyl hexyl benzoate), also referred to as DHHB, is an UV filter belonging to the group of benzophenone derivatives. It is sold under the tradename Uvinul A Plus by BASF. It is an UV-A filter.

4,4',4"-(1,3,5-Triazin-2,4,6-triyltriimino)tris-benzoic acid-tris(2-ethylhexyl)ester (INCI ethylhexyl triazone), also referred to as EHT, is an UV filter belonging to the group of triazine derivatives. It is sold under the tradename Uvinul T150 by BASF. It is an UV-B filter.

(RS)-2-Ethylhexyl-2-hydroxybenzoate (INCI ethylhexyl salicylate), also referred to as EHS, is an UV filter belonging to the group of salicylic derivatives. It is sold, e.g., under the tradename Neo Heliopan OS by Symrise. It is an UV-B filter.

In connection with the above sunscreen composition, it is to be understood that the sunscreen composition may comprise at least one additive as defined above, preferably at least one additive selected from the group consisting of emulsifier, emollients, viscosity regulators, stabilizers, preservatives, and combinations thereof.

In a preferred embodiment of the sunscreen composition as defined above, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 0.1 to 15 % by weight based on the total weight of the sunscreen composition.

In another preferred embodiment of the sunscreen composition as defined above, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 0.3 to 10 % by weight based on the total weight of the sunscreen composition.

In another preferred embodiment of the sunscreen composition as defined above, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 0.5 to 5 % by weight based on the total weight of the sunscreen composition.

In a particularly preferred embodiment of the sunscreen composition as defined above, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 1 to 3 % by weight based on the total weight of the sunscreen composition.

In an even more particularly preferred embodiment of the sunscreen composition as defined above, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 1 to 2.6 % by weight, based on the total weight of the sunscreen composition.

In connection with the above preferred embodiments, it is to be understood that the total weight of the sunscreen composition refers to the sunscreen composition as defined above, wherein the sunscreen composition may comprise at least one additive.

In a preferred embodiment of the sunscreen composition as defined above, the hexyl 2-[4-(diethylamino)-2-hydroxybenzoyl]benzoate (INCI diethylamino hydroxybenzoyl hexyl benzoate) is present in the sunscreen composition in an amount of from 0.1 to 10 % by weight based on the total weight of the sunscreen composition.

In a particularly preferred embodiment of the sunscreen composition as defined above, the hexyl 2-[4-(diethylamino)-2-hydroxybenzoyl]benzoate (INCI diethylamino hydroxybenzoyl hexyl benzoate) is present in the sunscreen composition in an amount of from 1 to 7 % by weight based on the total weight of the sunscreen composition.

In connection with the above preferred embodiments, it is to be understood that the total weight of the sunscreen composition refers to the sunscreen composition as defined above, wherein the sunscreen composition may comprise at least one additive.

In a preferred embodiment of the sunscreen composition as defined above, the 4,4',4"-(1,3,5-triazin-2,4,6-triyltriimino)tris-benzoic acid-tris(2-ethylhexyl)ester (INCI ethylhexyl triazone) is present in the sunscreen composition in an amount of from 0.1 to 5 % by weight based in the total weight of the sunscreen composition.

In a particularly preferred embodiment of the sunscreen composition as defined above, the 4,4',4"-(1,3,5-triazin-2,4,6-triyltriimino)tris-benzoic acid-tris(2-ethylhexyl)ester (INCI ethylhexyl triazone) is present in the sunscreen composition in an amount of from 0.5 to 3 % by weight based in the total weight of the sunscreen composition.

In connection with the above preferred embodiments, it is to be understood that the total weight of the sunscreen composition refers to the sunscreen composition as defined above, wherein the sunscreen composition may comprise at least one additive.

In a preferred embodiment of the sunscreen composition as defined above the (RS)-2-ethylhexyl-2-hydroxybenzoate (INCI ethylhexyl salicylate) is present in the sunscreen composition in an amount of from 0.1 to 5 % by weight based on the total weight of the sunscreen composition.

In connection with the above preferred embodiments, it is to be understood that the total weight of the sunscreen composition refers to the sunscreen composition as defined above, wherein the sunscreen composition may comprise at least one additive.

In a more preferred embodiment of the sunscreen composition as defined above, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 1 to 3 % by weight, the hexyl 2-[4-(diethylamino)-2-hydroxybenzoyl]benzoate (INCI diethylamino hydroxybenzoyl hexyl benzoate) is present in the sunscreen composition in an amount of from 1 to 7 % by weight, the 4,4',4"-(1,3,5-triazin-2,4,6-triyltriimino)tris-benzoic acid-tris(2-ethylhexyl)ester (INCI ethylhexyl triazone) is present in the sunscreen composition in an amount of from 0.5 to 3 % by weight and the (RS)-2-ethylhexyl-2-hydroxybenzoate (INCI ethylhexyl salicylate) is present in the sunscreen composition in an amount of from 0.1 to 5 % by weight based on the total weight of the sunscreen composition.

In connection with the above preferred embodiments, it is to be understood that the total weight of the sunscreen composition refers to the sunscreen composition as defined above, wherein the sunscreen composition may comprise at least one additive.

In another more preferred embodiment of the sunscreen composition as defined above, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 1 to 2.6 % by weight, the hexyl 2-[4-(diethylamino)-2-hydroxybenzoyl]benzoate (INCI diethylamino hydroxybenzoyl hexyl benzoate) is present in the sunscreen composition in an amount of from 1 to 7 % by weight, the 4,4',4"-(1,3,5-triazin-2,4,6-triyltriimino)tris-benzoic acid-tris(2-ethylhexyl)ester (INCI ethylhexyl triazone) is present in the sunscreen composition in an amount of from 0.5 to 3 % by weight and the (RS)-2-ethylhexyl-2-hydroxybenzoate (INCI ethylhexyl salicylate) is present in the sunscreen composition in an amount of from 0.1 to 5 % by weight based on the total weight of the sunscreen composition.

In connection with the above preferred embodiments, it is to be understood that the total weight of the sunscreen composition refers to the sunscreen composition as defined above, wherein the sunscreen composition may comprise at least one additive.

In a preferred embodiment of the sunscreen composition as defined above, the sunscreen composition comprises at least one further UV filter.

In another preferred embodiment of the sunscreen composition as defined above, the sunscreen composition comprises at least one further UV filter, wherein the at least one further UV filter is 2-phenyl-1H-benzimidazole-5-sulfonic acid (INCI phenylbenzimidazole sulfonic acid) and/or 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine).

In a particularly preferred embodiment of the sunscreen composition as defined above, the at least one further UV filter is 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine).

Thus, in a particularly preferred embodiment of the sunscreen composition as defined above, the sunscreen composition comprises at least one further UV filter, wherein the at least one further UV filter is preferably 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine).

In a particularly preferred embodiment of the sunscreen composition as defined above, the sunscreen composition comprises

(i) 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane),
(ii) hexyl 2-[4-(diethylamino)-2-hydroxybenzoyl]benzoate (INCI diethylamino hydroxybenzoyl hexyl benzoate),
(iii) 4,4',4"-(1,3,5-triazin-2,4,6-triyltriimino)tris-benzoic acid-tris(2-ethylhexyl)ester (INCI ethylhexyl triazone),
(iv) (RS)-2-ethylhexyl-2-hydroxybenzoate (INCI ethylhexyl salicylate), and
(v) 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine); and wherein the at least one further UV filter 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine) is present in the sunscreen composition in an amount of from 0.1 to 2.8 % by weight, based on the total weight of the sunscreen composition.

In another preferred embodiment of the sunscreen composition as defined above, the at least one further UV filter 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine) is present in the sunscreen composition in an amount of from 0.1 to 2.5 % by weight, based on the total weight of the sunscreen composition.

In a preferred embodiment of the sunscreen composition as defined above, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 0.1 to 15 % by weight based on the total weight of the sunscreen composition.

In another preferred embodiment of the sunscreen composition as defined above, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 0.3 to 10 % by weight based on the total weight of the sunscreen composition.

In another preferred embodiment of the sunscreen composition as defined above, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 0.5 to 5 % by weight based on the total weight of the sunscreen composition.

In a particularly preferred embodiment of the sunscreen composition as defined above, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 1 to 3 % by weight based on the total weight of the sunscreen composition.

In another particularly preferred embodiment of the sunscreen composition as defined above, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 1 to 2.6 % by weight, based on the total weight of the sunscreen composition.

In connection with the above preferred embodiments, it is to be understood that the total weight of the sunscreen composition refers to the sunscreen composition as defined above, wherein the sunscreen composition may comprise at least one additive.

In a preferred embodiment of the sunscreen composition as defined above, the hexyl 2-[4-(diethylamino)-2-hydroxybenzoyl]benzoate (INCI diethylamino hydroxybenzoyl hexyl benzoate) is present in the sunscreen composition in an amount of from 0.1 to 10 % by weight based on the total weight of the sunscreen composition.

In a particularly preferred embodiment of the sunscreen composition as defined above, the hexyl 2-[4-(diethylamino)-2-hydroxybenzoyl]benzoate (INCI diethylamino hydroxybenzoyl hexyl benzoate) is present in the sunscreen composition in an amount of from 1 to 7 % by weight based on the total weight of the sunscreen composition.

In connection with the above preferred embodiments, it is to be understood that the total weight of the sunscreen composition refers to the sunscreen composition as defined above, wherein the sunscreen composition may comprise at least one additive.

In a preferred embodiment of the sunscreen composition as defined above, the 4,4',4"-(1,3,5-triazin-2,4,6-triyltriimino)tris-benzoic acid-tris(2-ethylhexyl)ester (INCI ethylhexyl triazone) is present in the sunscreen composition in an amount of from 0.1 to 5 % by weight based in the total weight of the sunscreen composition.

In a particularly preferred embodiment of the sunscreen composition as defined above, the 4,4',4"-(1,3,5-triazin-2,4,6-triyltriimino)tris-benzoic acid-tris(2-ethylhexyl)ester (INCI ethylhexyl triazone) is present in the sunscreen composition in an amount of from 0.5 to 3 % by weight based in the total weight of the sunscreen composition.

In connection with the above preferred embodiments, it is to be understood that the total weight of the sunscreen composition refers to the sunscreen composition as defined above, wherein the sunscreen composition may comprise at least one additive.

In a preferred embodiment of the sunscreen composition as defined above the (RS)-2-ethylhexyl-2-hydroxybenzoate (INCI ethylhexyl salicylate) is present in the sunscreen composition in an amount of from 0.1 to 5 % by weight based on the total weight of the sunscreen composition.

In connection with the above preferred embodiments, it is to be understood that the total weight of the sunscreen composition refers to the sunscreen composition as defined above, wherein the sunscreen composition may comprise at least one additive.

In a more preferred embodiment of the sunscreen composition as defined above, the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 1 to 3 % by weight, the hexyl 2-[4-(diethylamino)-2-hydroxybenzoyl]benzoate (INCI diethylamino hydroxybenzoyl hexyl benzoate) is present in the sunscreen composition in an amount of from 1 to 7 % by weight, the 4,4',4"-(1,3,5-triazin-2,4,6-triyltriimino)tris-benzoic acid-tris(2-ethylhexyl)ester (INCI ethylhexyl triazone) is present in the sunscreen composition in an amount of from 0.5 to 3 % by weight, the (RS)-2-ethylhexyl-2-hydroxybenzoate (INCI ethylhexyl salicylate) is present in the sunscreen composition in an amount of from 0.1 to 5 % by weight and the 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine) is present in the sunscreen composition in an amount of from 0.1 to 2.5 % by weight based on the total weight of the sunscreen composition.

In connection with the above preferred embodiments, it is to be understood that the total weight of the sunscreen composition refers to the sunscreen composition as defined above, wherein the sunscreen composition may comprise at least one additive.

In connection with the above preferred embodiments it is to be understood that the hexyl 2-[4-(diethylamino)-2-hydroxybenzoyl]benzoate (INCI diethylamino hydroxybenzoyl hexyl benzoate) and the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) may be present in a certain ratio.

Thus, in a preferred embodiment of the sunscreen composition as defined above, the hexyl 2-[4-(diethylamino)-2-hydroxybenzoyl]benzoate (INCI diethylamino hydroxybenzoyl hexyl benzoate) and the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) are present in a ratio of larger or equal to 0.8.

The present invention is further illustrated by the following examples.

Examples

Process of manufacture of sunscreen compositions

The ingredients of part A, as well as the ingredients of part B as provided below in Tables 1.1.a, 1.1.b, 1.2.a, 1.2b, 1.3.a and 1.3.b for each tested sunscreen composition were combined and heated to 80 °C respectively, wherein part A was added to part B under stirring and was further homogenized. Subsequently, the sunscreen composition was cooled down to room temperature under stirring. If necessary, part C was added subsequently to adjust the pH.

Sunscreen compositions

The sunscreen compositions compared with each other in each case provide comparable SPF and/or UV-A protection. Only compositions of Tables 1.3 a and 1.3 b are part of the invention.

Table 1.1.a

| Ingredient (Trade Name) | Composition 1a Comparative Example | Composition 1b | Composition 1c |
|---|---|---|---|
| Part A | | | |
| Sucrose polystearate (and) hydrogenated polyisobutene (Emulgade Sucro Plus) | 3.00 | 3.00 | 3.00 |
| Disodium cetearyl sulfosuccinate (Emulgin Prisma) | 1.00 | 1.00 | 1.00 |
| Cetearyl alcohol (Lanette O) | 2.00 | 2.00 | 2.00 |
| $C_{12}$-$C_{15}$ alkyl benzoate (Cetiol AB) | 5.00 | 5.00 | 5.00 |
| Dibutyl Adipate (Cetiol B) | 11.00 | 11.00 | 11.00 |
| Phenoxyethanol and ethylhexylglycerine (Euxyl PE 9010) | 1.00 | 1.00 | 1.00 |
| DHHB (Uvinul A Plus) | 1.50 | - | 0.75 |
| DTS | - | 4.00 | 2.00 |
| Part B | | | |
| Water | 72.20 | 69.70 | 70.95 |
| Glycerine | 3.00 | 3.00 | 3.00 |
| Xanthan Gum (Rheocare XGN) | 0.30 | 0.30 | 0.30 |
| Total | 100.00 | 100.00 | 100.00 |
| UVA-PF | 3.7 | 3.9 | 3.6 |

Table 1.1.b

| Ingredient (Trade Name) | Composition 2a Comparative Example | Composition 2b |
|---|---|---|
| Part A | | |
| Sucrose polystearate (and) hydrogenated polyisobutene (Emulgade Sucro Plus) | 3.00 | 3.00 |
| Disodium cetearyl sulfosuccinate (Emulgin Prisma) | 1.00 | 1.00 |
| Cetearyl alcohol (Lanette O) | 2.00 | 2.00 |
| $C_{12}$-$C_{15}$ alkyl benzoate (Cetiol AB) | 5.00 | 5.00 |
| Dibutyl Adipate (Cetiol B) | 11.00 | 11.00 |

(continued)

| Ingredient (Trade Name) | Composition 2a Comparative Example | Composition 2b |
|---|---|---|
| Phenoxyethanol and ethylhexylglycerine (Euxyl PE 9010) | 1.00 | 1.00 |
| DHHB (Uvinul A Plus) | 2.00 | 1.00 |
| EHT (Uvinul T150) | 2.00 | 2.00 |
| EHS (NeoHeliopan OS) | 2.00 | 2.00 |
| DTS | - | 1.00 |
| Part B | | |
| Water | 67.70 | 67.70 |
| Glycerine | 3.00 | 3.00 |
| Xanthan Gum (Rheocare XGN) | 0.30 | 0.3 |
| Total | 100.00 | 100.00 |
| SPF | 11 | 11 |
| UVA-PF | 4.7 | 4.7 |

Table 1.2.a

| Ingredient (Trade Name) | Composition 3a Comparative Example | Composition 3b | Composition 3c |
|---|---|---|---|
| Part A | | | |
| Sucrose polystearate (and) hydrogenated polyisobutene (Emulgade Sucro Plus) | 3.00 | 3.00 | 3.00 |
| Disodium cetearyl sulfosuccinate (Emulgin Prisma) | 1.00 | 1.00 | 1.00 |
| Cetearyl alcohol (Lanette O) | 2.00 | 2.00 | 2.00 |
| $C_{12}$-$C_{15}$ alkyl benzoate (Cetiol AB) | 5.00 | 5.00 | 5.00 |
| Dibutyl Adipate (Cetiol B) | 11.00 | 11.00 | 11.00 |
| Phenoxyethanol and ethylhexylglycerine (Euxyl PE 9010) | 1.00 | 1.00 | 1.00 |
| BEMT (Tinosorb S) | 1.50 | - | 0.75 |
| DTS | - | 3.00 | 1.50 |
| Part B | | | |
| Water | 72.20 | 70.70 | 71.45 |
| Glycerine | 3.00 | 3.00 | 3.00 |
| Xanthan Gum (Rheocare XGN) | 0.30 | 0.30 | 0.30 |
| Total | 100.00 | 100.00 | 100.00 |
| SPF | 4 | 4 | 4 |
| UVA-PF | 3.5 | 3.3 | 3.4 |

15

Table 1.2.b

| Ingredient (Trade Name) | Composition 4a Comparative Example | Composition 4b |
|---|---|---|
| Part A | | |
| Sucrose polystearate (and) hydrogenated polyisobutene (Emulgade Sucro Plus) | 3.00 | 3.00 |
| Disodium cetearyl sulfosuccinate (Emulgin Prisma) | 1.00 | 1.00 |
| Cetearyl alcohol (Lanette O) | 2.00 | 2.00 |
| $C_{12}$-$C_{15}$ alkyl benzoate (Cetiol AB) | 5.00 | 5.00 |
| Dibutyl Adipate (Cetiol B) | 11.00 | 11.00 |
| Phenoxyethanol and ethylhexylglycerine (Euxyl PE 9010) | 1.00 | 1.00 |
| BEMT (Tinosorb S) | 2.00 | - |
| DHHB (Uvinul A Plus) | 2.00 | 2.00 |
| EHT (Uvinul T150) | 2.50 | 2.50 |
| EHS (NeoHeliopan OS) | 5.00 | 5.00 |
| DTS | - | 4.00 |
| Part B | | |
| Water | 62.20 | 60.20 |
| Glycerine | 3.00 | 3.00 |
| Xanthan Gum (Rheocare XGN) | 0.30 | 0.3 |
| Total | 100.00 | 100.00 |
| SPF | 21 | 26 |
| UVA-PF | 7.4 | 9.6 |

Table 1.3.a

| Ingredient (Trade Name) | Composition 5a Comparative Example | Composition 5b |
|---|---|---|
| Part A | | |
| PEG-100 Glyceryl Stearate, Glyceryl Stearate (Emulgade 165) | 3.00 | 3.00 |
| Sodium Stearoyl Glutamate (Emulgin SG) | 1.00 | 1.00 |
| Stearic Acid, Palmitic Acid (Cutina FS 45) | 2.00 | 2.00 |
| C12-15 Alkyl Benzoate (Cetiol AB) | 5.00 | 5.00 |
| Phenoxyethanol and Ethylhexylglycerin (Euxyl PE 9010) | 1.00 | 1.00 |
| Homosalate (NeoHeliopan HMS) | 10.00 | 10.00 |
| EHS (NeoHeliopan OS) | 5.00 | 5.00 |
| DHHB (Uvinul A Plus) | 3.00 | 3.00 |
| EHT (Uvinul T150) | 3.00 | 3.00 |
| BEMT (Tinosorb S) | 4.00 | 2.70 |
| DTS | 5.00 | 7.50 |
| Part B | | |
| Water | Qsp 100 % | Qsp 100 % |
| Glycerin | 3.00 | 3.00 |

(continued)

| Ingredient (Trade Name) | Composition 5a Comparative Example | Composition 5b |
|---|---|---|
| Acrylates/C10-30 Alkyl Acrylate Crosspolymer (Pemulen TR-2) | 0.30 | 0.30 |
| TDSA (33% solution, Solfilter TDSA) | 3.03 (1% active) | 3.03 (1% active) |
| Part C | | |
| Triethanolamine | qs | qs |
| Total | 100.00 | 100.00 |
| SPF | 48 | 47 |
| UVA-PF | 11 | 11 |

Table 1.3.b

| Ingredient (Trade Name) | Composition 6a Comparative Example | Composition 6b |
|---|---|---|
| Part A | | |
| PEG-100 Glyceryl Stearate, Glyceryl Stearate (Emulgade 165) | 3.00 | 3.00 |
| Sodium Stearoyl Glutamate (Emulgin SG) | 1.00 | 1.00 |
| Stearic Acid, Palmitic Acid (Cutina FS 45) | 2.00 | 2.00 |
| C12-15 Alkyl Benzoate (Cetiol AB) | 5.00 | 5.00 |
| Phenoxyethanol and Ethylhexylglycerin (Euxyl PE 9010) | 1.00 | 1.00 |
| Homosalate (NeoHeliopan HMS) | 2.00 | 2.00 |
| EHS (NeoHeliopan OS) | 5.00 | 5.00 |
| DHHB (Uvinul A Plus) | 2.00 | 2.00 |
| EHT (Uvinul T150) | 2.50 | 2.50 |
| BEMT (Tinosorb S) | 3.00 | 2.00 |
| DTS | 2.80 | 4.50 |
| Part B | | |
| Water | Qsp 100 % | Qsp 100 % |
| Propylene Glycol | 3.00 | 3.00 |
| Acrylates/C10-30 Alkyl Acrylate Crosspolymer (Pemulen TR-2) | 0.30 | 0.30 |
| TDSA (33% solution, Solfilter TDSA) | 1.00 (0.3% active) | 1.00 (0.3% active) |
| Part C | | |
| Triethanolamine | qs | qs |
| Total | 100.00 | 100.00 |
| SPF | 30 | 47 |
| UVA-PF | 11 | 11 |

Measurement of fabric staining

Fabric staining of sunscreen compositions was measured by the L*a*b as described above. The measurement was performed with a spectrophotometer using the following instrument: Remission Spectrometer Datacolor SF 400 (light D65, observer 10°).

The difference between the color of a sunscreen composition stain on a white textile and the color of the white textile alone was determined following the assumption that $\Delta E^* = \Delta b^*$ in order to determine fabric staining. The lower the color difference, the lower is the fabric staining.

Further, the difference between the color of a sunscreen composition stain on a white textile after 1 or 3 washing cycles and the color of the white textile alone was determined following the assumption that $\Delta E^* = \Delta b^*$ in order to determine the washability. The lower the color difference in comparison to the value obtained before washing, the more has fabric staining been reduced, i.e. the better the washability of the sunscreen composition from textiles.

For applying the sunscreen compositions in order to make a stain on a white textile, the sunscreen composition was diluted in water or ethanol at a concentration of 10 % by weight based on the total weight of the diluted mixture. 300 mg ($15\ mg/cm^2$) of the diluted mixture is applied on a white textile (whiteness adjusted to WG 170 $\pm$ 10, after washing WG 190 $\pm$ 10) in order to obtain a stain.

For determining the washability, 1 or 3 washing cycles were performed in a washing machine using a temperature of 40 °C for 20 minutes and Persil Universal Gel obtained from Henkel (3 g on stain). Each textile was washed separately.

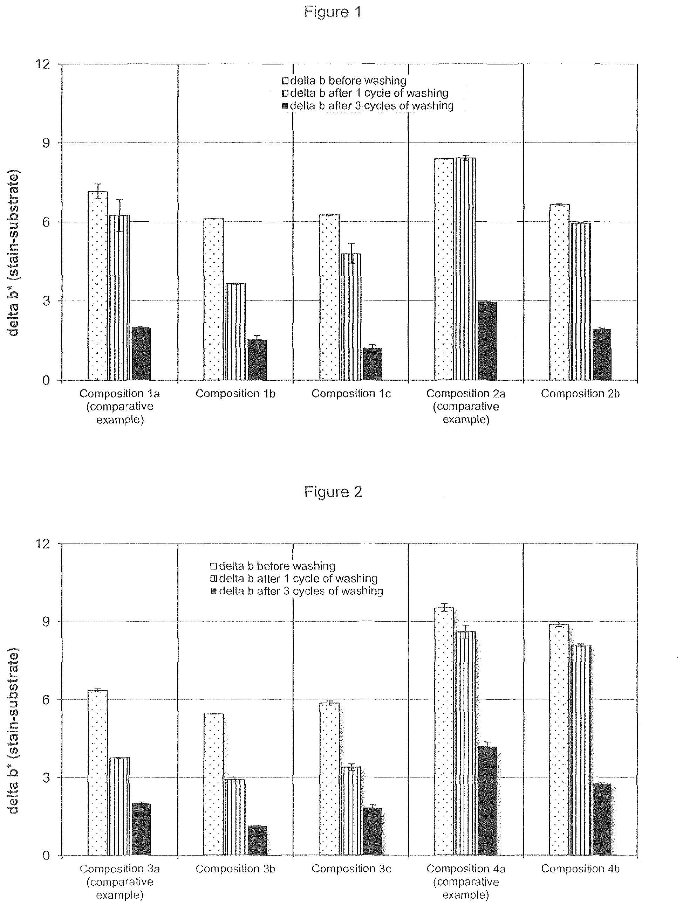

The results for sunscreen compositions 1a, 1b, 1c, 2a and 2b are shown in Table 2.1.a, 2.1.b and Figure 1.

Table 2.1.a

|  | Composition 1a Comparative Example | Composition 1b | Composition 1c |
|---|---|---|---|
| $\Delta b^*$ (before washing) | 7.15 | 6.11 | 6.25 |
| SD (before washing) | 0.28 | 0.01 | 0.03 |
| $\Delta b^*$ (after 1 cycle) | 6.24 | 3.65 | 4.79 |
| SD (after 1 cycle) | 0.61 | 0.02 | 0.38 |
| $\Delta b^*$ (after 3 cycles) | 2 | 1.54 | 1.22 |
| SD (after 3 cycles) | 0.04 | 0.14 | 0.11 |

Table 2.1.b

|  | Composition 2a Comparative Example | Composition 2b |
|---|---|---|
| $\Delta b^*$ (before washing) | 8.37 | 6.64 |
| SD (before washing) | 0 | 0.04 |
| $\Delta b^*$ (after 1 cycle) | 8.41 | 5.95 |
| SD (after 1 cycle) | 0.1 | 0.03 |
| $\Delta b^*$ (after 3 cycles) | 2.97 | 1.94 |
| SD (after 3 cycles) | 0.03 | 0.03 |

The results of the sunscreen compositions 3a, 3b, 3c, 4a and 4b are shown in Table 2.2.a, 2.2.b and Figure 2.

Table 2.2.a

|  | Composition 3a Comparative Example | Composition 3b | Composition 3c |
|---|---|---|---|
| $\Delta b^*$ (before washing) | 6.36 | 5.45 | 5.86 |
| SD (before washing) | 0.06 | 0.01 | 0.08 |
| $\Delta b^*$ (after 1 cycle) | 3.75 | 2.92 | 3.39 |
| SD (after 1 cycle) | 0.02 | 0.09 | 0.13 |
| $\Delta b^*$ (after 3 cycle) | 2 | 1.15 | 1.83 |
| SD (after 3 cycle) | 0.05 | 0 | 0.11 |

Table 2.2.b

|  | Composition 4a Comparative Example | Composition 4b |
|---|---|---|
| $\Delta b^*$ (before washing) | 9.53 | 8.89 |
| SD (before washing) | 0.16 | 0.09 |

(continued)

|  | Composition 4a Comparative Example | Composition 4b |
|---|---|---|
| $\Delta b^*$ (after 1 cycle) | 8.59 | 8.09 |
| SD (after 1 cycle) | 0.25 | 0.05 |
| $\Delta b^*$ (after 3 cycle) | 4.2 | 2.77 |
| SD (after 3 cycle) | 0.16 | 0.05 |

The results of the sunscreen compositions 5a, 5b, 6a and 6b are shown in Table 2.3.a and 2.3.b.

Table 2.3.a

|  | Composition 5a Comparative Example | Composition 5b |
|---|---|---|
| $\Delta b^*$ (before washing) | 12.7 | 12 |
| SD (before washing) | 0.04 | 0.02 |
| $\Delta b^*$ (after 1 cycle) | 8.7 | 8 |
| SD (after 1 cycle) | 0.12 | 0 |
| $\Delta b^*$ (after 3 cycle) | 3.4 | 2.8 |
| SD (after 3 cycle) | 0.05 | 0.06 |

Table 2.3.b

|  | Composition 6a Comparative Example | Composition 6b |
|---|---|---|
| $\Delta b^*$ (before washing) | 11 | 11 |
| SD (before washing) | 0.07 | 0.03 |
| $\Delta b^*$ (after 1 cycle) | 7 | 6.6 |
| SD (after 1 cycle) | 0.23 | 0.12 |
| $\Delta b^*$ (after 3 cycle) | 3 | 2.5 |
| SD (after 3 cycle) | 0.08 | 0.07 |

**Claims**

1. Use of 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) to reduce fabric staining of sunscreen compositions.

2. Use of 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) to facilitate the washability of sunscreen compositions from textiles.

3. The use according to claim 1 or 2, wherein the sunscreen composition has an UV-A protection, which is at least one third of the sun protection factor of the sunscreen composition, and/or shows a critical wavelength of at least 370 nm.

4. The use according to any one of claims 1 to 3, wherein the sunscreen composition comprises at least one further UV-A filter.

5. The use according to claim 4, wherein the at least one further UV-A filter is selected from the group consisting of triazine derivatives, benzotriazole derivatives, benzophenone derivatives, dibenzoylmethane derivatives, camphor derivatives and combinations thereof.

6. The use according to claim 4 or 5, wherein the at least one further UV-A filter is selected from the group consisting of 4-(tert.-butyl)-4'-methoxydibenzoylmethane (INCI butyl methoxydibenzoylmethane), hexyl 2-[4-(diethylamino)-2-hydroxybenzoyl]benzoate (INCI diethylamino hydroxybenzoyl hexyl benzoate), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), and combinations thereof.

7. The use according to any one of claims 1 to 6, wherein the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 0.2 to 15 % by weight, preferably from 0.5 to 5 % by weight, more preferably from 1 to 3 % by weight based on the total weight of the sunscreen composition.

8. The use according to any one of claims 1 to 7, wherein the sunscreen composition further comprises at least one particulate UV filter, wherein preferably the at least one particulate UV filter is an organic particulate UV filter selected from the group consisting 2,2'-methylene bis[6-(2H-benzotriazol-2-yl)-4-(1,1,3,3-tetramethylbutyl)phenol] (INCI methylene bis-benzotriazolyl tetramethylbutylphenol), 2,4,6-tris(biphenyl-4-yl)-1,3,5-triazine (INCI trisbiphenyl triazine), 1,1'-(1,4-piperazinediyl)bis[1-[2-[4-(diethylamino)-2-hydroxybenzoyl]phenyl]-methanone (INCI bis-(di-ethylaminohydroxybenzoyl benzoyl) piperazine), 5,6,5',6'-tetraphenyl-3-3'-(1,4-phenylene)bis(1,2,4-triazine) (INCI phenylene bis-diphenyltriazine), and combinations thereof, an inorganic particulate UV filter is selected from the group consisting of titanium dioxide, zinc oxide, and combinations thereof, or a combination of any of the foregoing organic and inorganic particulate UV filters.

9. The use according to any one of claims 1 to 7, wherein the sunscreen composition further comprises at least one water soluble UV filter selected from the group consisting of [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1-bicyclo[2.2.1]heptanyl]methanesulfonic acid (INCI terephthalylidene dicamphor sulfonic acid), 2-phenyl-1H-benzimidazole-5-sulfonic acid (INCI phenylbenzimidazole sulfonic acid), disodium phenyl dibenzimidazole tetrasulfonate, and combinations thereof.

10. The use according to claim 1 to 9, wherein the sunscreen composition further comprises at least one UV-filter, wherein the at least one further UV-B filter is selected from the group consisting of 4,4',4"-(1,3,5-triazin-2,4,6-triyltriimino)tris-benzoic acid-tris(2-ethylhexyl)ester (INCI ethylhexyl triazone), (RS)-2-ethylhexyl-2-hydroxybenzoate (INCI ethylhexyl salicylate), 3,3,5-trimethyl-cyclohexyl salicylate (homosalate), 2-ethylhexyl 2-cyano-3,3-diphenyl-2-propenoate (INCI octocrylene), (RS)-2-ethylhexyl-(2E)-3-(4-methoxyphenyl)prop-2-enoate (INCI ethylhexyl methoxycinnamate), 4-methoxycinnamic acid 2-ethylhexyl ester (INCI ethylhexyl methoxycinnamate), malonate derivatives such as dimethicone diethyl benzalmalonate, diethylhexyl butamido triazone, and combinations thereof.

11. A sunscreen composition comprising

(i) 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane),
(ii) hexyl 2-[4-(diethylamino)-2-hydroxybenzoyl]benzoate (INCI diethylamino hydroxybenzoyl hexyl benzoate),
(iii) 4,4',4"-(1,3,5-triazin-2,4,6-triyltriimino)tris-benzoic acid-tris(2-ethylhexyl)ester (INCI ethylhexyl triazone), and
(iv) (RS)-2-ethylhexyl-2-hydroxybenzoate (INCI ethylhexyl salicylate);
wherein the sunscreen composition comprises at least one further UV filter, wherein the at least one further UV filter is 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine); and
wherein the at least one further UV filter 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine) is present in the sunscreen composition in an amount of from 0.1 to 2.8 % by weight, based on the total weight of the sunscreen composition.

12. The sunscreen composition according to claim 11, wherein the 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane) is present in the sunscreen composition in an amount of from 0.1 to 15 % by weight.

13. The sunscreen composition according to claim 11 or 12, wherein the hexyl 2-[4-(diethylamino)-2-hydroxybenzoyl]benzoate (INCI diethylamino hydroxybenzoyl hexyl benzoate) is present in an amount of from 0.1 to 10 % by weight.

14. The sunscreen composition according to any one of claims 11 to 13, wherein the 4,4',4"-(1,3,5-triazin-2,4,6-triyltriimino)tris-benzoic acid-tris(2-ethylhexyl)ester (INCI ethylhexyl triazone) is present in an amount of from 0.1 to 5 % by weight, preferably from 0.5 to 3 % by weight based on the total weight of the sunscreen composition.

**15.** The sunscreen composition according to any one of claims 11 to 14, wherein the (RS)-2-ethylhexyl-2-hydroxybenzoate (INCI ethylhexyl salicylate) is present in an amount of from 0.1 to 5 % by weight based on the total weight of the sunscreen composition.

**Patentansprüche**

**1.** Verwendung von 2-(2H-Benzotriazol-2-yl)-4-methyl-6-[2-methyl-3-[1,3,3,3-tetramethyl-1-[(trimethylsilyl)oxy]-1-disiloxanyl]propyl]phenol (INCI Drometrizoltrisiloxan) zum Reduzieren der Stoffeinfärbung durch Sonnenschutzzusammensetzungen.

**2.** Verwendung von 2-(2H-Benzotriazol-2-yl)-4-methyl-6-[2-methyl-3-[1,3,3,3-tetramethyl-1-[(trimethylsilyl)oxy]-1-disiloxanyl]propyl]phenol (INCI Drometrizoltrisiloxan) zum Erleichtern der Auswaschbarkeit von Sonnenschutzzusammensetzungen aus Textilien.

**3.** Verwendung nach Anspruch 1 oder 2, wobei die Sonnenschutzzusammensetzung einen UV-A-Schutz aufweist, der mindestens ein Drittel des Sonnenschutzfaktors der Sonnenschutzzusammensetzung beträgt und/oder eine kritische Wellenlänge von mindestens 370 nm aufweist.

**4.** Verwendung nach einem der Ansprüche 1 bis 3, wobei die Sonnenschutzzusammensetzung mindestens einen weiteren UV-A-Filter umfasst.

**5.** Verwendung nach Anspruch 4, wobei der mindestens eine weitere UV-A-Filter ausgewählt ist aus der Gruppe bestehend aus Triazin-Derivaten, Benzotriazol-Derivaten, Benzophenon-Derivaten, Dibenzoylmethan-Derivaten, Kampfer-Derivaten und Kombinationen davon.

**6.** Verwendung nach Anspruch 4 oder 5, wobei der mindestens eine weitere UV-A-Filter ausgewählt ist aus der Gruppe bestehend aus 4-(tert-Butyl)-4'-methoxydibenzoylmethan (INCI Butylmethoxydibenzoylmethan), Hexyl-2-[4-(diethylamino)-2-hydroxybenzoyl]benzoat (INCI Diethylaminohydroxybenzoylhexylbenzoat), 2,4-Bis-{[4-(2-ethylhexyloxy)-2-hydroxy]phenyl}-6-(4-methoxyphenyl)-1,3,5-triazin (INCI Bisethylhexyloxyphenolmethoxyphenyltriazin) und Kombinationen davon.

**7.** Verwendung nach einem der Ansprüche 1 bis 6, wobei das 2-(2H-Benzotriazol-2-yl)-4-methyl-6-[2-methyl-3-[1,3,3,3-tetramethyl-1-[(trimethylsilyl)oxy]-1-disiloxanyl]propyl]phenol (INCI Drometrizoltrisiloxan) in der Sonnenschutzzusammensetzung in einer Menge von 0,2 bis 15 Gew.-%, vorzugsweise von 0,5 bis 5 Gew.-%, besonders bevorzugt von 1 bis 3 Gew.-%, bezogen auf das Gesamtgewicht der Sonnenschutzzusammensetzung, vorhanden ist.

**8.** Verwendung nach einem der Ansprüche 1 bis 7, wobei die Sonnenschutzzusammensetzung weiter mindestens einen UV-Partikelfilter umfasst, wobei der mindestens eine UV-Partikelfilter vorzugsweise ein organischer UV-Partikelfilter, der ausgewählt ist aus der Gruppe bestehend aus 2,2'-Methylenbis[6-(2H-benzotriazol-2-yl)-4-(1,1,3,3-tetramethylbutyl)phenol] (INCI Methylenbisbenzotriazolyltetramethylbutylphenol), 2,4,6-Tris(biphenyl-4-yl)-1,3,5-triazin (INCI Trisbiphenyltriazin), 1,1'-(1,4-Piperazindiyl)bis[1-[2-[4-(diethylamino)-2-hydroxybenzoyl]phenyl]-methanon (INCI Bis(diethylaminohydroxybenzoylbenzoyl)piperazin), 5,6,5',6'-Tetraphenyl-3-3'-(1,4-phenylen)bis(1,2,4-triazin) (INCI Phenylenbisdiphenyltriazin) und Kombinationen davon, ein anorganischer UV-Partikelfilter, der ausgewählt ist aus der Gruppe bestehend aus Titandioxid, Zinkoxid und Kombinationen davon, oder eine Kombination beliebiger der vorstehenden organischen und anorganischen UV-Partikelfilter ist.

**9.** Verwendung nach einem der Ansprüche 1 bis 7, wobei die Sonnenschutzzusammensetzung weiter mindestens einen wasserlöslichen UV-Filter umfasst, der ausgewählt ist aus der Gruppe bestehend aus [(3Z)-3-[[4-[(Z)-[7,7-Dimethyl-2-oxo-1-(sulfomethyl)-3-bicyclo[2.2.1]heptanyliden]methyl]phenyl]methyliden]-7,7-dimethyl-2-oxo-1-bicyclo[2.2.1]heptanyl]methansulfonsäure (INCI Terephthalylidendikampfersulfonsäure), 2-Phenyl-1H-benzimidazol-5-sulfonsäure (INCI Phenylbenzimidazolsulfonsäure), Dinatriumphenyldibenzimidazoltetrasulfonat und Kombinationen davon.

**10.** Verwendung nach Anspruch 1 bis 9, wobei die Sonnenschutzzusammensetzung weiter mindestens einen UV-Filter umfasst, wobei der mindestens eine weitere UV-B-Filter ausgewählt ist aus der Gruppe bestehend aus 4,4',4"-(1,3,5-Triazin-2,4,6-triyltriimino)tris-benzoesäure-tris(2-ethylhexyl)ester (INCI Ethylhexyltriazon), (RS)-2-Ethylhexyl-2-hydroxybenzoat (INCI Ethylhexylsalicylat), 3,3,5-Trimethylcyclohexylsalicylat (Homosalat), 2-Ethylhexyl-2-cyano-3,3-diphenyl-2-propenoat (INCI Octocrylen), (RS)-2-Ethylhexyl-(2E)-3-(4-methoxyphenyl)prop-2-enoat (INCI Ethylhexylmethoxycinnamat), 4-Methoxyzimtsäure-2-ethylhexylester (INCI Ethylhexylmethoxycinnamat), Malonat-Derivaten wie Dimethicondiethylbenzalmalonat, Diethylhexylbutamidotriazon und Kombinationen davon.

11. Sonnenschutzzusammensetzung, umfassend

(i) 2-(2H-Benzotriazol-2-yl)-4-methyl-6-[2-methyl-3-[1,3,3,3-tetramethyl-1-[(trimethylsilyl)oxy]-1-disiloxanyl]propyl]phenol (INCI Drometrizoltrisiloxan),

(ii) Hexyl-2-[4-(diethylamino)-2-hydroxybenzoyl]benzoat (INCI Diethylaminohydroxybenzoylhexylbenzoat),

(iii) 4,4',4"-(1,3,5-Triazin-2,4,6-triyltriimino)tris-benzoesäure-tris(2-ethylhexyl)ester (INCI Ethylhexyltriazon) und

(iv) (RS)-2-Ethylhexyl-2-hydroxybenzoat (INCI Ethylhexylsalicylat);

wobei die Sonnenschutzzusammensetzung mindestens einen weiteren UV-Filter umfasst, wobei der mindestens eine weitere UV-Filter 2,4-Bis-{[4-(2-ethylhexyloxy)-2-hydroxy]phenyl}-6-(4-methoxyphenyl)-1,3,5-triazin (INCI Bisethylhexyloxyphenolmethoxyphenyltriazin) ist; und

wobei der mindestens eine weitere UV-Filter 2,4-Bis-{[4-(2-ethylhexyloxy)-2-hydroxy]phenyl}-6-(4-methoxyphenyl)-1,3,5-triazin (INCI Bisethylhexyloxyphenolmethoxyphenyltriazin) in der Sonnenschutzzusammensetzung in einer Menge von 0,1 bis 2,8 Gew.-%, bezogen auf das Gesamtgewicht der Sonnenschutzzusammensetzung, vorhanden ist.

12. Sonnenschutzzusammensetzung nach Anspruch 11, wobei das 2-(2H-Benzotriazol-2-yl)-4-methyl-6-[2-methyl-3-[1,3,3,3-tetramethyl-1-[(trimethylsilyl)oxy]-1-disiloxanyl]propyl]phenol (INCI Drometrizoltrisiloxan) in der Sonnenschutzzusammensetzung in einer Menge von 0,1 bis 15 Gew.-% vorhanden ist.

13. Sonnenschutzzusammensetzung nach Anspruch 11 oder 12, wobei das Hexyl-2-[4-(diethylamino)-2-hydroxybenzoyl]benzoat (INCI-Diethylaminohydroxybenzoylhexylbenzoat) in einer Menge von 0,1 bis 10 Gew.-% vorhanden ist.

14. Sonnenschutzzusammensetzung nach einem der Ansprüche 11 bis 13, wobei der 4,4',4"-(1,3,5-Triazin-2,4,6-triyltriimino)trisbenzoesäuretris(2-ethylhexyl)ester (INCI Ethylhexyltriazon) in einer Menge von 0,1 bis 5 Gew.-%, vorzugsweise in einer Menge von 0,5 bis 3 Gew.-%, bezogen auf das Gesamtgewicht der Sonnenschutzzusammensetzung, vorhanden ist.

15. Sonnenschutzzusammensetzung nach einem der Ansprüche 11 bis 14, wobei das (RS)-2-Ethylhexyl-2-hydroxybenzoat (INCI Ethylhexylsalicylat) in einer Menge von 0,1 bis 5 Gew.-%, bezogen auf das Gesamtgewicht der Sonnenschutzzusammensetzung, vorhanden ist.

**Revendications**

1. Utilisation de 2-(2H-benzotriazol-2-yl)-4-méthyl-6-[2-méthyl-3-[1,3,3,3-tétraméthyl-1-[(triméthylsilyl)oxy]-1-disiloxanyl]propyl]phénol (INCI drometrizole trisiloxane) pour réduire la coloration des tissus de compositions d'écran solaire.

2. Utilisation de 2-(2H-benzotriazol-2-yl)-4-méthyl-6-[2-méthyl-3-[1,3,3,3-tétraméthyl-1-[(triméthylsilyl)oxy]-1-disiloxanyl]propyl]phénol (INCI drometrizole trisiloxane) pour faciliter la lessivabilité de compositions d'écran solaire de textiles.

3. Utilisation selon la revendication 1 ou 2, dans laquelle la composition d'écran solaire présente une protection contre les UV-A qui est d'au moins un tiers du facteur de protection solaire de la composition d'écran solaire et/ou présente une longueur d'onde critique d'au moins 370 nm.

4. Utilisation selon l'une quelconque des revendications 1 à 3, la composition comprenant au moins un autre filtre UV-A.

5. Utilisation selon la revendication 4, dans laquelle l'au moins un autre filtre UV-A est choisi dans le groupe constitué par les dérivés de triazine, les dérivés de benzotriazole, les dérivés de benzophénone, les dérivés de dibenzoylméthane, les dérivés de camphre et leurs combinaisons.

6. Utilisation selon la revendication 4 ou 5, dans laquelle l'au moins un autre filtre UV-A est choisi dans le groupe constitué par 4-(tert.-butyl)-4'-méthoxydibenzoylméthane (INCI butyl methoxydibenzoylmethane), 2-[4-(diéthylamino)-2-hydroxybenzoyl]benzoate d'hexyle (INCI diethylamino hydroxybenzoyl hexyl benzoate), 2,4-bis-{[4-(2-éthyl-hexyloxy)-2-hydroxy]-phényl}-6-(4-méthoxyphenyl)-1,3,5-triazine (INCI bis-éthylhexyloxyphénol méthoxyphényl triazine) et leurs combinaisons.

7. Utilisation selon l'une quelconque des revendications 1 à 6, dans laquelle le 2-(2H-benzotriazol-2-yl)-4-méthyl-6-[2-méthyl-3-[1,3,3,3-tétraméthyl-1-[(triméthylsilyl)oxy]-1-disiloxanyl]propyl]phénol (INCI drometrizole trisiloxane) est présent dans la composition solaire en une quantité de 0,2 à 15 % en poids, de préférence de 0,5 à 5 % en poids, plus préférablement de 1 à 3 % en poids sur la base du poids total de la composition d'écran solaire.

8. Utilisation selon l'une quelconque des revendications 1 à 7, dans laquelle la composition d'écran solaire comprend en outre au moins un filtre UV particulaire, dans laquelle de préférence l'au moins un filtre UV particulaire est un filtre UV particulaire organique choisi dans le groupe constitué par 2,2'-méthylène bis[6-(2H-benzotriazol-2-yl)-4-(1,1,3,3-tétraméthylbutyl)phénol] (INCI methylene bis-benzotriazolyl tetramethylbutylphenol), 2,4,6-tris(biphényl-4-yl)-1,3,5-triazine (INCI trisbiphenyl triazine), 1,1'-(1,4-pipérazinediyl)bis[1-[2-[4-(diéthylamino)-2-hydroxybenzoyl]phényl]-méthanone (INCI bis-(diethylaminohydroxybenzoyl benzoyl) piperazine), 5,6,5',6'-tétraphényl-3-3'-(1,4-phénylène)bis(1,2,4-triazine) (INCI phenylene bis-diphenyltriazine), et leurs combinaisons, un filtre UV particulaire inorganique est choisi dans le groupe constitué par le dioxyde de titane, l'oxyde de zinc et leurs combinaisons, ou une combinaison de quelconques des filtres UV particulaires organiques ou inorganiques mentionnés précédemment.

9. Utilisation selon l'une quelconque des revendications 1 à 7, dans laquelle la composition d'écran solaire comprend en outre au moins un filtre UV soluble dans l'eau choisi dans le groupe constitué par acide [(3Z)-3-[[4-[(Z)-[7,7-diméthyl-2-oxo-1-(sulfométhyl)-3-bicyclo[2.2.1]heptanylidène]méthyl]phényl]méthylidène]-7,7-diméthyl-2-oxo-1-bicyclo[2.2.1]heptanyl]méthanesulfonique (INCI terephthalylidene dicamphor sulfonic acid), acide 2-phényl-1H-benzimidazole-5-sulfonique (INCI phenylbenzimidazole sulfonic acid), tétrasulfonate de phényldibenzimidazole disodique et leurs combinaisons.

10. Utilisation selon les revendications 1 à 9, dans laquelle la composition d'écran solaire comprend en outre au moins un filtre UV, dans laquelle l'au moins un autre filtre UV-B est choisi dans le groupe constitué par ester de tris(2-éthylhexyle) d'acide 4,4',4"-(1,3,5-triazin-2,4,6-triyltriimino)tris-benzoïque (INCI ethylhexyl triazone), (RS)-2-hydroxybenzoate de 2-éthylhexyle (INCI ethylhexyl salicylate), 3,3,5-triméthyl-cyclohexyl salicylate (homosalate), 2-cyano-3,3-diphényl-2-propénoate de 2-éthylhexyle (INCI octocrylene), (RS)-(2E)-3-(4-méthoxyphényl)prop-2-énoate de 2-éthylhexyle (INCI ethylhexyl methoxycinnamate), ester de 2-éthylhexyle d'acide 4-méthoxycinnamique (INCI ethylhexyl methoxycinnamate), dérivés de malonate tels que le benzalmalonate de diéthyle diméthicone, la diéthylhexylbutamidotriazone, et leurs combinaisons.

11. Composition d'écran solaire comprenant

(i) 2-(2H-benzotriazol-2-yl)-4-méthyl-6-[2-méthyl-3-[1,3,3,3-tétraméthyl-1-[(triméthylsilyl)oxy]-1-disiloxanyl]propyl]phénol (INCI drometrizole trisiloxane),
(ii) 2-[4-(diéthylamino)-2-hydroxybenzoyl]benzoate d'hexyle (INCI diethylamino hydroxybenzoyl hexyl benzoate),
(iii) ester de tris(2-éthylhexyle) d'acide 4,4',4"-(1,3,5-triazin-2,4,6-triyltriimino)tris-benzoïque (INCI ethylhexyl triazone), et
(iv) 2-hydroxybenzoate de 2-éthylhexyle (RS) (INCI salicylate d'éthylhexyle) ;

dans laquelle la composition d'écran solaire comprend au moins un autre filtre UV, ledit au moins un autre filtre UV étant la 2,4-bis-{[4-(2-éthyl-hexyloxy)-2-hydroxy]-phényl}-6-(4-méthoxyphényl)-1,3,5 triazine (INCI bis-éthylhexyloxyphénol méthoxyphényl triazine) ; et l'au moins un autre filtre UV 2,4-bis-{[4-(2-éthyl-hexyloxy)-2-hydroxy]-phényl}-6-(4-méthoxyphényl)-1,3,5 triazine (INCI bis-ethylhexyloxyphenol methoxyphenyl triazine) étant présent dans la composition d'écran solaire en une quantité allant de 0,1 à 2,8 % en poids, sur la base du poids total de la composition d'écran solaire.

12. Composition d'écran solaire selon la revendication 11, dans laquelle le 2-(2H-benzotriazol-2-yl)-4-méthyl-6-[2-méthyl-3-[1,3,3,3-tétraméthyl-1-[(triméthylsilyl)oxy]-1-disiloxanyl]propyl]phénol (INCI drometrizole trisiloxane) est présent dans la composition d'écran solaire en une quantité de 0,1 à 15 % en poids.

13. Composition d'écran solaire selon la revendication 11 ou 12, dans laquelle le 2-[4-(diéthyl-lamino)-2-hydroxybenzoyl]benzoate d'hexyle (INCI diéthylamino hydroxybenzoyl hexyl benzoate) est présent en une quantité de 0,1 à 10 % en poids.

14. Composition d'écran solaire selon l'une quelconque des revendications 11 à 13, dans laquelle l'ester de tris (2-éthylhexyle) d'acide 4,4',4"-(1,3,5-triazine-2,4,6-triyltriimino)tris-benzoïque (INCI ethylhexyl triazone) est présent en une quantité allant de 0,1 % à 5 % en poids, préférablement de 0,5 à 3 % en poids, sur la base du poids total de la composition d'écran solaire.

15. Composition d'écran solaire selon l'une quelconque des revendications 11 à 14, dans laquelle le (RS)-2-hydroxybenzoate de 2-éthylhexyle (INCI ethylhexyl salicylate) est présent en une quantité de 0,1 à 5 % en poids sur la base du poids total de la composition d'écran solaire.

Figure 1

Figure 2

**EP 3 860 555 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102014207919 A1
- DE 102014207916 A1
- DE 102014207935 A1
- DE 102014207924 A1
- EP 2939710 A1
- EP 3150190 A1
- EP 3153151 A1
- EP 3150258 A1
- EP 3150189 A
- EP 3354253 A2